# EUROPEAN PATENT APPLICATION

(11) **EP 4 344 619 A1**
(43) Date of publication of application: **03.04.2024**
(21) Application number: 22198679.7
(22) Date of filing: 29.09.2022
(51) Int. Cl.: A61B 5/00, H01L 21/50, H01L 23/10, A61F 2/01, B01D 61/18, B01D 63/00, B01D 63/08, A61B 5/0215, A61B 5/145, G01N 27/327, G01N 33/487, B23K 26/03

(54) **LASER WELDED ENCLOSURE FOR ELECTRONICS, CIRCUITRY OR SENSORS**

(71) Applicant: SCHOTT AG, 55122 Mainz (DE); Schott Primoceler Oy, 33720 Tampere (FI)
(72) Inventor: THOMAS, Jens Ulrich, 55128 Mainz (DE); MÄÄTTÄNEN, Antti, 33710 Tampere (FI); OTT, Christian, 84539 Ampfing (DE)
(74) Representative: Blumbach · Zinngrebe Patentanwälte PartG mbB

(57) **Abstract**

An enclosure is shown, comprising at least a first substrate and a second substrate, wherein the first substrate is transparent at least in part and/or at least for a wavelength bandwidth, at least one common laser weld zone wherein material of the first and second substrates is mixed, wherein the laser weld zone is designed such to permanently join the first substrate to the second substrate and/or to provide for a hermetic sealing between the first substrate and the second substrate, at least one function zone, for example comprising a cavity, enclosed inside the enclosure, and at least one fluid channel allowing for a fluid flow between said function zone and an outside.

## Description

### Field of the invention

The invention is related to an enclosure, for example, for providing an at least partially hermetically sealed compartment or cavity in at least two layers of substrates, an immersible sensor element, an immersible donator element and an immersible specific filter element.

### Background and Summary of the invention

Enclosures can be used, for example, to protect electronics, circuitry or sensors. It is possible to use hermetically sealed implementations of aforementioned enclosures for medical implants, for example, in a therapy to cure a heart disease, or, for example, in a retina or for any type of bio-processor. Known are bio-processors, which are made from titan. It is principally known to provide several parts or layers and to arrange them such that in an inner region components can be situated. For example European Patent EP 3 012 059 B1 shows a method for manufacturing a transparent component for protecting an optical component. A new laser welding method is used therein.

In some use cases that serve as one of several problems to be solved by the present invention it is desirable to measure or influence one or several properties of the fluid or material surrounding the enclosure. Sensors can be used or implanted by means of the invention, for example, for use in particular rough or (chemically) aggressive environmental conditions. Further examples for usage of the invention are Micro-Electro-Mechanic-Systems (MEMS), but also a pressure sensor, blood gas sensor, a glucose meter, such as a blood glucose meter, or the like.

Further fields of concern for the present invention, where the enclosure has been designed to accomplish at least one of these problems, might include providing a medical treatment and/or a medical treatment device for diseases or corporal malfunctions of animals or human beings; or it might comprise a standardized leaking container for certification of product lines; or it might comprise a humidity sensors.

Further fields of usage for the present invention can be found in augmented reality, such as non-fogging waveguide assemblies; or in home appliance/consumer fields such as scent dispensers; or in automotive such as gas sensors. The present invention can also be designed to work as a purificaiton device for water or other liquids, or gases. For example, the invention may also be used in the scope of electro mobility; But also in aviation and space environment, in high temperature environments and in the field of micro optics.

The aforementioned applications concern devices faced with rough environmental conditions and which thus have to be designed especially robust or protected from these conditions - and/or they concern biological interaction with the enclosure.

Further, the invention may, as one option of a solution to the problems, allow to some extent an exchange, such as a fluid exchange, with the surroundings where the enclosure is implemented.

Therefore, and to summarize, it is an object of the invention to provide a means capable of interacting with, changing or measuring properties of a fluid, and ideally small enough to be placed into the same fluid.

In a particular interesting embodiment and to explain one showcase of the invention the possibility to dispense a dosage of a fluid such as a medicament or a chemical substance over time to the surroundings of the enclosure may be provided, such as insulin, e.g. for the treatment of diabetes. The surroundings of the enclosure are hereinafter referred to as outside. In the same embodiment and/or at the same time the blood sugar level could be measured to derive the amount of insulin needed over time. Advantageously, in the same embodiment a depleted enclosure could communicate or send a signal.

The object of the invention is achieved by subject matter of the independent claims. Preferred embodiments of the invention are subject of the dependent claims.

An enclosure according to the invention comprises at least a first substrate and a second substrate, wherein the first substrate is transparent at least in part and/or at least for a wavelength bandwidth. The first substrate may be having an outer surface and the second substrate may also be having an outer surface. The first substrate and the second substrate are typically arranged next to each other in such a way that an inner surface of the first substrate is adjacent to an inner surface of the second substrate. In other words, in this example the two substrates are arranged next to each other, i.e., in direct proximity to one another, wherein the inner surface plane of the first substrate is in contact with the inner surface plane of the second substrate. Typically, each substrate of the enclosure is comprised rather flat, in the meaning that its lateral dimensions are far greater than its width. The outer surface as well as the inner surface opposing the outer surface comprise comparably longer dimensions than the circumferential rim, which lies in between the outer surfaces. By aligning several, at least two, substrates next to or above one another, a stack of substrates is formed. Such a stack of substrates may then be laser-welded in order to firmly join the substrates with each other. For example, for each two substrates, one laser weld line may be used for joining these two substrates. Therefore, for example, in the case that four substrates shall be used to form the enclosure, at least three laser weld lines may be introduced, where each laser weld line is arranged such to join the respectively two neighboring substrates of the substrate stack.

The top most substrate, preferably comprises said transparent material and/or is transparent at least in part of its surface or volume and/or at least for a bandwidth of wavelengths. Whenever the laser is used for welding and joining the substrates to each other, the laser therefore can pass through the top most substrate in order to e.g. reach the interface zone in between the two substrates to be welded. It is referred to the top-most substrate as the laser is typically shot into the substrate(s) from above. It is clear that reference is made to that substrate through which the laser has to pass in order to arrive at the laser spot where it is intended to be positioned. So in a setup where it is feasible to shoot the laser from the side or from below, then the reference to the "top-most substrate" may no longer be applicable, but the "top most substrate" typically refers to the substrate which, during laser welding, is closest to the laser source. In any case, it is preferred, that the substrate material (substrate volume) through which the laser travels in order to place the laser spot inside the substrate / substrate stack, comprises transparent material and/or is transparent at least in part of its surface or volume and/or at least for a bandwidth of wavelengths, typically the bandwidth of the laser used for welding.

The enclosure therefore comprises at least one common laser weld zone wherein material of the first and second substrates is mixed, wherein the laser weld zone is designed such to permanently join the first substrate to the second substrate and/or to provide for a hermetic sealing between the first substrate and the second substrate. Such a at least one laser weld zone extends from within the first substrate to within the second substrate and permanently joins the first substrate to the second substrate. The laser weld zone may further be designed to provide hermetical seal to a function zone situated such that it is surrounded by the laser weld zone. But on the other side, also additional laser weld line(s) may be provided to introduce, assure or improve bonding and/or hermetic seal of a function zone.

For example, the laser weld zone could comprise a first separation spacing S1 to the outer surface of the first substrate and a second separation spacing S2 to the outer surface of the second substrate. If separation spacings S1 and S2 are applied, the laser weld zone may be fully enclosed inside the enclosure. This means, in other words, that the laser weld zone is fully enclosed within the enclosure without touching or penetrating either outer surface of the first or second substrate. So to say, the laser weld zone is fully enclosed with material from either the first substrate or the second substrate, or any substrate comprised in the enclosure. The enclosure therefore fully encloses the laser weld zone, which is not in contact with the environment surrounding the enclosure. This does also mean that neither the first outer surface nor the second outer surface, which is the outer surface of the second substrate, is deteriorated by the at least one laser weld zone. This may increase mechanical and/or chemical resistance of the enclosure. It may also be advantageous if the laser weld zone is initially fully surrounded by material of the enclosure, e.g. including separation spacings S1 and S2, but the enclosure may, e.g. in a later process step, be thinned down so that the laser weld zone is exposed to the surrounding. E.g. the laser weld zone may then also form part of the first outer surface and/or of the second outer surface. Reference is made in this respect, and in particular with respect to methods of polishing down one of the substrates of an enclosure, to German Patent Application DE 10 2020 117 194.3, which is hereby incorporated in its entirety into the present application.

The laser weld zone can be a portion of the first substrate and/or it may be a portion of the second substrate. Preferably, the laser weld zone comprises material from the first substrate as well as material from the second substrate. In other words, material from the first substrate is melted and at the same time material from the second substrate is melted, and the materials are mixed with each other. The laser weld zone may further comprise a refractive index that differs from a refractive index of non-welded portion of the first and/or second substrate.

The enclosure according to the invention further comprises at least one function zone, for example comprising a cavity, enclosed inside the enclosure. Said function zone may be circumferentially enclosed in the enclosure. Such a cavity may further comprise one or more devices or components to be enclosed inside the enclosure. The function zone may also comprise some detector means, some microelectronic or mechanical system and/or any micro optics. Even an energy-generating device like a battery or an energy-harvesting device such as a small solar cell may be provided in the function zone. The function zone can comprise a coating, for example situated in between the two substrates or provided on one of the inner sides of the substrates. The cavity may, for example, be arranged in the plane of the first and/or second substrate. By way of example, the cavity may be excavated from the first substrate and/or from the second substrate, for example by means of an abrasive method. In the case that three substrates are used to provide the enclosure, the uppermost and lowermost substrate may also be continuous substrates, where the function zone or cavity is arranged in the plane of the intermediate substrate. For example, the intermediate substrate can comprise holes, which become the cavity, when the three substrates are stacked on one another.

The enclosure further comprises at least one fluid channel allowing for a fluid flow between said function zone and an outside. Such a fluid channel allows for direct contact and/or exchange of (parts of) the inside of the enclosure with the surrounding fluid, allowing for interaction of the functional means inside the cavity with properties of the surrounding. For example, it might allow for an inflow of fluid into the cavity and thus for analysis of its properties, and/or it might allow for an outflow of fluid into the surrounding and thus for influencing properties of the surrounding fluid.

In a particular interesting design the at least one fluid channel may function like a gate that is defined by the quantity of fluid passing through it. In particular, the passage of fluid through the at least one channel might be regulated by a channel regulator.

In an improvement of the invention the first substrate can comprise glass, glass ceramics, plastics or crystal such as sapphire. Alternatively or additionally the second substrate and/or an intermediate layer may comprise glass, glass ceramics, plastics, metal, silicon, or crystal such as sapphire. But also a material such as paper, porous glass, e.g. CoralPor^{®} from Schott AG, could be used e.g. as a filter element as the second substrate and/or an intermediate layer.

Additionally or alternatively said laser weld zone is arranged in a welding plane such that the welding plane cuts the laser weld zone e.g. centrical.

Said at least one fluid channel may be defined by the laser weld zone, for example by a shape or extension dimension of the laser weld zone. Said fluid channel may, for example, constitute a trench in the laser weld zone.

Said at least one fluid channel may be arranged in the welding plane. Said at least one fluid channel may further comprise a height h in a direction perpendicular to said welding plane, which is smaller or equal to a height HL of the welding zone in a direction perpendicular to its connecting plane. The laser weld zone may also be situated with a height HL1 inside the first substrate and with a height HL2 = h - HL1 inside the second substrate. In other words, the complete laser weld line height HL may be situated inside either the first substrate or the second substrate, which means that no additional material or spacing is present in between the first substrate and the second substrate. This indicates that the first substrate is directly bonded and mixed with the second substrate by means of the laser weld process. When the laser weld line is equally reaching into both substrates, HL1 = HL2, but, for example, if the laser weld zone and/or laser weld line is shot deeper into the first substrate, then HL1 is greater than HL2. Still, the sum of HL1 + HL2 preferably sums up to HL.

The at least one laser weld zone and/or laser weld line may circumference the function zone in a distance DF, where the distance may correspond, for example, to the height HL or less. The distance DF may also correspond to double the height HL or less.

Said at least one fluid channel may further comprise a flat or mostly rectangular shape. Further, said at least one fluid channel may comprise one or several ridge(s). Said at least one fluid channel may also be provided at opposing sides of the enclosure, for example one channel at every side of the enclosure. Said at least one fluid channel can be provided in the form of one or more nanovoid(s).

Said cavity may preferably be interconnected with said at least one fluid channel. Further, a second cavity may be provided, wherein the second cavity is connected to a second fluid channel. The first cavity may be connected to the second cavity by means of a passage. Said passage may preferably comprise a passage diameter and wherein the passage diameter is smaller than the diameter of the fluid channel and/or the second fluid channel.

The at least one channel may comprise said height (h), for example in a direction perpendicular to said welding plane, of 10 nm or more, preferably 25 nm or more, further preferably 50 nm or more, further preferably 100 nm or more, or 300 nm or more, or even 500 nm or more. Said height (h) of the at least one channel may also be, for example in a direction perpendicular to said welding plane, 1000 nm or less, preferably 750 nm or less, further preferably 500 nm or less, further preferably 250 nm or less, or even 100 nm or less. The at least one channel may further comprise a width (b), for example in said welding plane, of at least 5 nm or more, further of at least 20 nm or more, preferably of 100 nm or more, further preferably of 1 µm or more, or even of 10 µm or more, or even 100 µm or more. Said width (b) may also be 2 mm or less, further 500 µm or less, preferably 200 µm or less, further preferably 50 µm or less. The flow rate of the channels depends of the fluid and its (temperature dependent) viscosity as well as the wall roughness through the capillary effect; We characterize the channels with the helium leakage test, because of the following reasons:
- It is a non-destructive test.
- It is an inert gas
- It is a reference substance more independent against influencing factors (e.g. atmospheric moisture, molecular interactions).
- Its flow rate is less temperature dependent
   We found that channels with helium leakage rate in the following intervals to exhibit beneficial flowing rates also for other fluids and gasses:
   a helium leakage rate of 1x10-8 (mbar × liter)/s or more, preferably of 1x10-6 (mbar × liter)/s or more, and/or provides for a fluid flow rate of 5x10-6 (mbar × liter)/s or less,
   or 1x10-4 (mbar × liter)/s or less.

The enclosure may, in an improvement of the invention, further comprise a pressure difference generating means, such as an expandable or shrinkable reservoir, an electromechanical pressing means, a chemical potential gradient source or a micro pump.

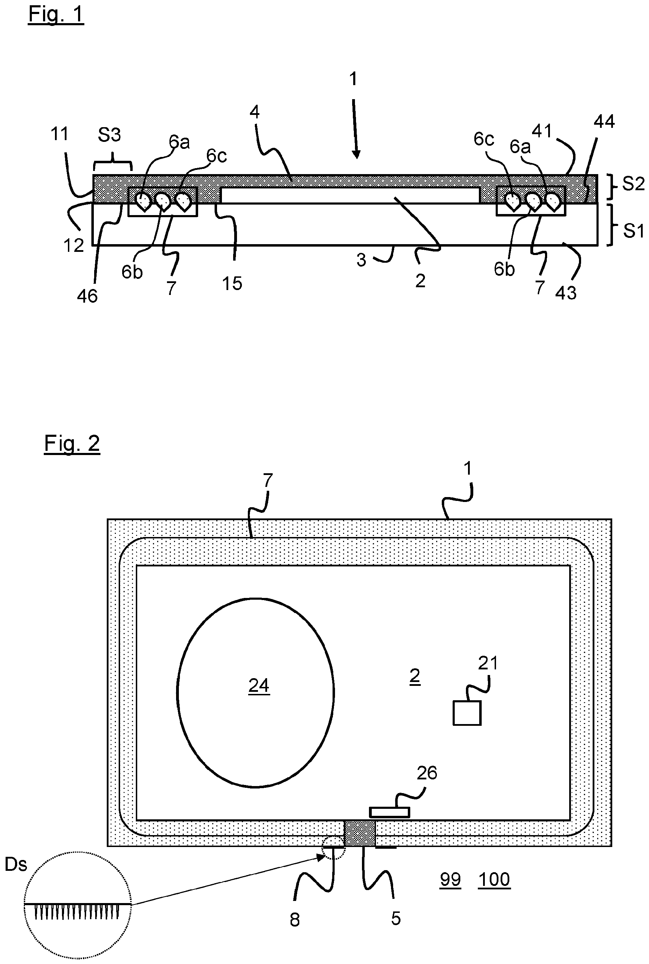

Further, the enclosure may be comprising an ingrow preventing surface situated next to or around the at least one opening, the ingrow preventing surface, for example, comprising a biologically incompatible material and/or a smoothened or spiked surface, see e.g. detail Ds in Figure 2. Spikes of a spiked surface may be used as micro structuring, which counteracts the adhesion of cells, especially because spikes can penetrate the cells of bacteria, for example, as a biocidal surface.

The enclosure may also further comprise a fluid property sensor arranged on the outside of the enclosure to be exposed in full fluid contact. Additionally or alternatively, it may further comprise at least one electrical connection from the function zone to the outside of the enclosure.

The at least one laser weld zone may extend from within the first substrate to within the second substrate so that material from the first substrate is mixed into the second substrate and/or material from the second substrate is mixed into the first substrate to permanently and directly join the first substrate to the second substrate. The at least one laser weld zone may further comprise a first separation spacing S1 to the outer surface of the first substrate and a second separation spacing S2 to the outer surface of the second substrate. In the laser weld zone may a convection zone be present, where material from the first substrate is mixed with material from the second substrate. Further, the laser weld zone could be a portion of the first and/or second substrate having a refractive index that differs from a refractive index of a non-welded portion of the first and/or second substrate.

The laser weld zone is preferably arranged around said function zone, for example circumferentially around said function zone. The laser weld zone may also be arranged such, that it comprises a third separation spacing S3 to the at least one channel or opening. At least one functional component may be arranged in said cavity, such as an electronics component, a MEMS or a MOEMS.

An enclosure as depicted hereinabove may be used for example as a sensor element, a wafer level packaged component, a micro lens compound, a micro optical chip, or an LED or laser diode device. Such an enclosure may in an embodiment also be used as a pharma packaging, a pharmaceutical product emitter, or a blood property sensor.

In the scope of the present disclosure there is further an immersible sensor element for measuring fluid properties, comprising at least a first substrate and a second substrate, wherein the first substrate is transparent at least in part and/or at least for a wavelength bandwidth, at least one common laser weld zone wherein material of the first and second substrates is mixed , wherein the laser weld zone is designed such to permanently join the first substrate to the second substrate and/or to provide for a hermetic sealing between the first substrate and the second substrate, at least one function zone, for example comprising a cavity, enclosed inside the enclosure, and comprising a fluid sensor element, and at least one fluid channel allowing for a fluid flow between said function zone and an outside. A fluid property to be measured might include blood pressure or blood glycose level. The fluid also may be, for example liquor, lymphatic fluid, lacrimal fluid, a glandular secret as e.g. insulin, urine or an other body fluid.

Significantly, also extracoporal analysis of blood sugar concentration may be obtained by analyzing lacrimal fluid with the inventive immersible sensor element providing for an extracoporal blood glycose sensor which is not invasive to a patient's body.

In the scope of the present disclosure there is further an immersible donator element for changing or adapting fluid properties, comprising at least a first substrate and a second substrate, wherein the first substrate is transparent at least in part and/or at least for a wavelength bandwidth, at least one common laser weld zone wherein material of the first and second substrates is mixed, wherein the laser weld zone is designed such to permanently join the first substrate to the second substrate and/or to provide for a hermetic sealing between the first substrate and the second substrate, at least one function zone, for example comprising a cavity, enclosed inside the enclosure, and comprising a donator fluid reservoir, and at least one fluid channel allowing for a fluid flow between said function zone and an outside. The donator element may be designed for emitting a pharmaceutical product.

Such an immersible donator may further comprise a controllable or regulated valve as a flow regulator for adjusting the amount and/or time of pharmaceutical product to be emitted.

In the scope of the present disclosure there is also an immersible specific filter element for being deployed in a fluid, comprising at least a first substrate and a further substrate, wherein the first substrate is transparent at least in part and/or at least for a wavelength bandwidth, at least one common laser weld zone wherein material of the first and further substrates is mixed, wherein the laser weld zone is designed such to permanently join the first substrate to the further substrate and/or to provide for an at least partially hermetic sealing between the first substrate and the further substrate, at least one function zone comprising a cavity, and at least a first fluid channel allowing for a fluid inflow into said function zone from an outside, and at least a second fluid channel allowing for a fluid outflow into said outside, wherein the second fluid channel is designed such that selectable particles or components of the fluid are retained inside or outside the cavity when said fluid streams through said filter element. The fluid may be, for example, blood, liquor, lymphatic fluid, lacrimal fluid, a glandular secret as e.g. insulin, urine or an other body fluid.

The fluid also may be a fluid phameceutical substance.

The respective fluid may contain corpuscular elements as blood cells or other body cells or may contain particles as for instance particles of pharmaceutical substances e.g. particles having a defined solubility allowing for a defined application of sustained release pharmaceuticals.

The laser weld zone can be designed in such a way that it at least partially hermetically seals the function zone in the enclosure. For example, a laser weld line may be drawn without gap around the function zone, so that the first substrate is hermetically joined to the second substrate. The laser weld zone typically is formed by a series of laser dots in a sequence. These laser dots may be placed close enough to each other, so that multiple laser dots overlap spatially. While the first laser pulses generate the absorption zone, absorption of subsequent pulses heat up the absorption zone and enlarge it toward the laser beam. This heat accumulation in combination with the translation of the sample generates a continuous laser weld line. Such a continuous laser weld line comprises hermetically sealing properties, when it is drawn completely around the function zone. However, according to the invention there is at least one fluid channel allowing for a fluid flow between said function zone and an outside of the enclosure. This arrangement comprising hermetically sealing the cavity by means of the laser weld zone and providing a fluid interconnection between the inside or interior of the cavity and the outside or exterior of the enclosure, e.g. by means of a channel, is termed an at least partially hermetically sealed cavity or an at least partially hermetically sealed enclosure. By means of the laser weld line, the substrates are firmly joined with each other in that the laser weld line melts or mixes the material of the first substrate with material of the second substrate in a "convection zone", in that the laser dot is placed such that the aforementioned mixture of materials is achieved. In other words, the convection zone is characterized in that material from the first substrate is mixed with material from the second substrate in the convection zone.

The laser weld zone may also be arranged such that it comprises a third separation spacing S3 to the circumferential rim of the enclosure. In other words, the laser weld zone leaves a secure spacing to the circumferential rim so that also said rim is not interfered by the laser weld zone. This spacing may be called a "tolerance zone". Typically, the enclosure is diced out of a wafer or a waferstack comprising a plurality of arrangements including each an at least first substrate, a second substrate and preferably one or more intermediate substrates, each arranged in a stack, respectively, so that additional spacing in between the laser weld zone and the rim may be advantageous in that the laser weld zone is not interfered upon dicing the enclosure out of the waferstack.

The invention is described in more detail and in view of preferred embodiments hereinafter. Reference is made to the attached drawings wherein like numerals have been applied to like or similar components.

### Brief Description of the Figures

It is shown in
- Fig. 1: a schematic cross-sectional side view of an enclosure,
- Fig. 2 to 4: schematic top views of embodiments of enclosures,
- Figs. 5 to 9: schematic cross-sectional side views of embodiments of enclosures,
- Figs. 10 and 11: an immersible donator element,
- Figs. 12 to 14: an immersible specific filter element.

### Detailed Description of the Invention

Figure 1 shows a cross-sectional view of an embodiment of an enclosure 1, where a bottom layer 3 is arranged underneath a top layer 4. The bottom layer or first substrate 3 and the top layer or second substrate 4 are at least partially hermetically bonded to each other by means of three laser weld lines 6a, 6b, 6c forming the laser weld zone 7. The laser weld zone 7 stretches into material of the first substrate 3 as well as into material of the second substrate 4, thus mixing the materials of the two substrates 3, 4. In introducing a firm seal, the laser weld zone 7 may provide an at least partially hermetically sealed area comprising a function zone 2, where one or several laser weld lines 6a, 6b, 6c may be drawn around such an area 2, except for the channel 5.. The two substrates 3, 4 contact each other at the inner side 46 of the first substrate 3 and the inner side 44 of the second substrate 4. Both inner sides 44, 46 touch each other at the contact area 15. On the side of the enclosure 1 a spacing S3 is provided in between the edge 11 of the enclosure 1 defining a rim 12 and the laser weld zone 7. In the perspective of Figure 1, the rim 12 is orientated vertically, whereas the planes 41, 43, 44 and 46 of the first and second substrates 3, 4 are orientated horizontally. The rim 12 may connect the outer side 41 of the second substrate 4 with the outer side 43 of the first substrate 3, so that the total outer surface area may be comprised by the outer side 41 of the second substrate, the rim 12 and the outer side 43 of the first substrate 3.

Referring to Figure 2, a top perspective view of an enclosure 1 is shown, where laser weld zone 7 is provided partially surrounding function zone 2. A channel 5 interconnects the surrounding outside 99 with the inner side of the function zone 2, e.g. a cavity. So a fluid interconnection between the outside 99 and a cavity inside an enclosure 1 is provided. For example, by means of said channel 5 fluid properties of the outside 99 may be measured, and/or fluid may be donated from the inside to the outside, and/or fluid may be exchanged between the outside 99 and the cavity 2 on the inner side of the enclosure 1. The cavity 2 is preferably hermetically sealed by means of the laser weld zone 7 to all sides except for the channel 5, so that for instance the leakage rate and/or fluid interchangeability can be defined by defining the properties, shape and/or dimensions of channel 5.

Channel 5 may be provided with spikes and other surface properties as additional or alternative ingrow preventing surface 8 already during fabrication, e.g. with laser ablation, which at least counteracts the adhesion of cells. The surface modification can be limited to areas, e.g. entrance areas, so as not to impair the general flow properties.

Further, as depicted in Fig. 2, a reservoir 24 is shown where the reservoir 24 might serve, according to the purpose for the enclosure 1, as e.g. a pressure regulator or as a fluid reservoir for providing a donator fluid to the cavity 2 or via channel 5 to the outside 99. Functional element 21 is shown schematically in the enclosure. A regulating means 26 is also shown schematically which could regulate the inflow or outflow, e.g. by means of a slider for narrowing down the width of channel 5 and such for influencing the possible flow rate through channel 5. Regulating means 26 could also be provided in the form of a soluble barrier which, upon contact with the surrounding fluid at the outside 99, dissolves and allows for fluid exchange. The elements 21, 24, 26 such as the depicted reservoir 24 and regulating means 26 may be provided independently from each other in a cavity 2 of an enclosure 1, but are provided in one common figure for sake of conciseness.

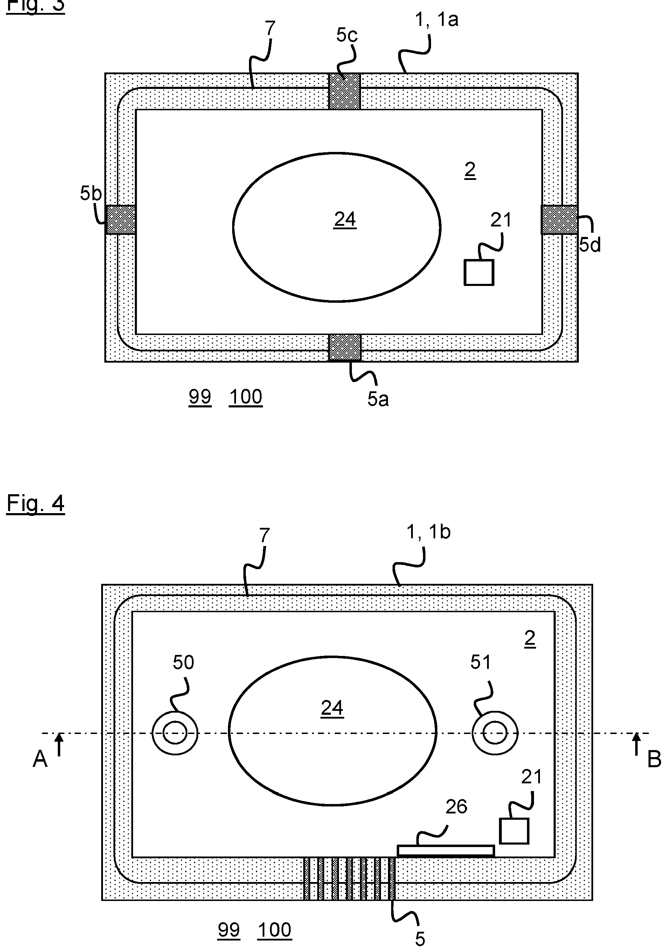

Turning now to Fig. 3 an enclosure 1 is shown with several channels 5a, 5b, 5c, 5d evenly distributed around the rim 12 of the enclosure 1. Again a reservoir 24 and a functional element 21 is shown inside the function zone 2, however depending on the purpose of the enclosure 1 they may be present or not. By providing several channels 5 the leakage rate may be increased. By distributing the channels 5 e.g. on each side of the enclosure 1 an improved fluid flow distribution may be achieved. In particular, the embodiment shown in Fig. 3 can also provide a cross-flow through the enclosure 1 or a fluid exchange, e.g. wherein influx of fluid is allowed via channel 5a, and wherein outflux is allowed via channels 5b, 5c and 5d. Flow rate may be self-regulated or might be regulated by means of functional element 21.

When viewing Fig. 4 channel 5 is provided as several leakage trenches such as micro vias. Such an embodiment may allow for increase of leakage rate in comparison with the embodiment of Fig. 2, but at the same time limit the channel diameter and/or allow for selection of particles that are small enough to pass through channel 5.

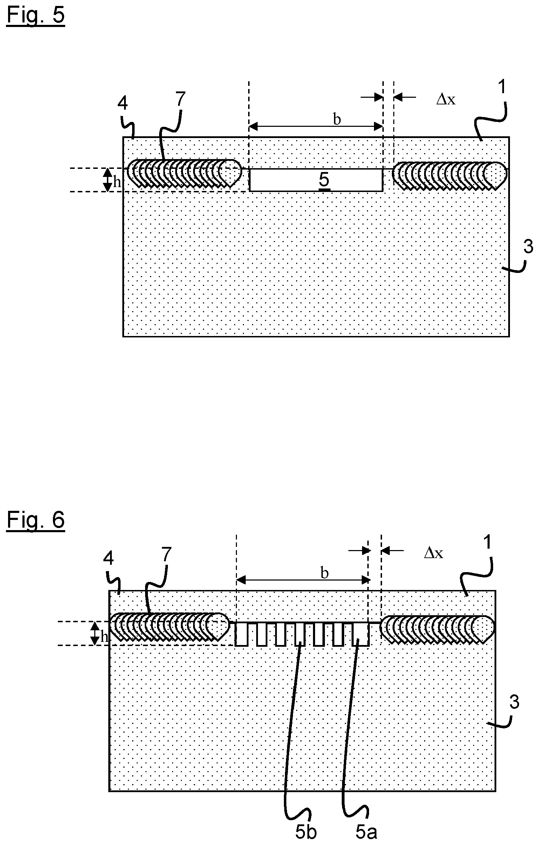

In relation to figures 5 through 9 several designs of channels 5 are discussed with their respective potential making and use. Fig. 5 shows a box-like trench 5 in the plane of the laser weld zone 7, having width b and a lateral spacing Δx. In the making of channel 5 as depicted in Fig. 5 a small shallow trench 5 may be elaborated from the base substrate 3, e.g. by means of ultrashort pulse laser ablation. The height h of the trench may be set in the order of 50 nm up to 1 µm, preferably 300 nm ± 150 nm. The width b of the trench may be set, by way of example, in the interval between 0 mm up to 2 mm. When both substrates 3, 4 are composed of glass and a glass-to-glass enclosure 1 is used the width may preferably be in between 10 to 20 µm, for example 15 ±10 µm. Further as an example, b + 2 Δx ≥ w, with w being the width of the laser weld zone 7 (e.g. 40 µm). Herein, Δx is a spacing between the last "shot" of the laser weld line and the beginning of the channel or trench 5. After elaboration of the trench 5 the top substrate 4 may be placed on top of base substrate 3 and contact bonded. The laser weld zone 7 is shot at the interface between top and bottom substrate 3, 4, wherein it stops at a distance Δx before the boundaries of the trench 5 or part-channels 5a, 5b ,,,. Since without the trench, the uninterrupted laser weld zone 7 would provide for a hermeticity for the cavity 2, the overall leakage only depends on the properties of the channel 5, like its geometry. Thus an enclosure with a defined leakage rate is provided.

Instead of using laser ablation, the channel 5 may also be elaborated by means of etching, scratching or grinding the rim 12, and/or at least part of the material of one of the substrates 3, 4, 4a may be provided porous, i.e. may comprise a filter material, such as paper, porous glass, e.g. CoralPor^{®} from Schott AG, providing an intrinsic leak rate in the porous material. The channel 5 can be fabricated by a lithography process e.g. in case of silicon. In case of at least one substrate comprising metal or consisting of metal the metal surface may be prepared or provided slightly rough or porous. A polishing pattern may be used which results in a non-flat inner surface of the substrate 3, 4. Also a part of the substrate 3, 4 could be cut out to elaborate the channel 5 in one of the substrates 3, 4.

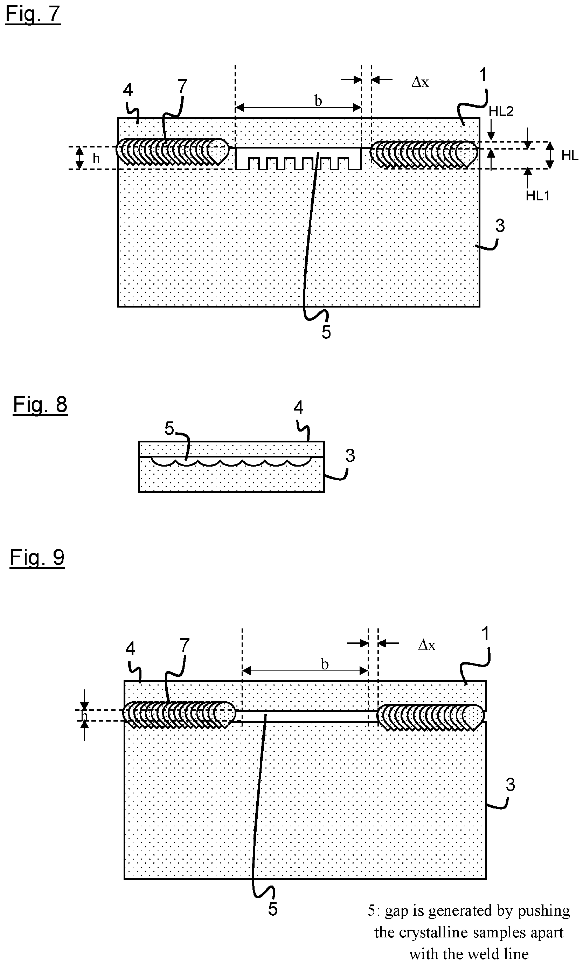

Referring to Fig. 6 a comb-like channel 5 with several part-channels 5a, 5b, ... is shown, similar to the embodiment shown in Fig. 4. In general, the channel 5 does not have to be homogeneous, it can also be a saw tooth or any other shape with a defined, open cross-section A; it can also be a "lift-off" by the welding lines. Fig. 7 shows another embodiment, where the comb-like structure of the channel 5 is not continuously from bottom to top, but affects only part of the channel 5. An upper part of the channel 5 is shaped continuously, whereas a lower part of the channel 5 is comb-like. Fig. 8a shows a variation where the structure of the channel 5 is shaped by truncations. For example, this form could be obtained by means of drilling.

In yet another example as shown in Fig. 9 an observation obtained from laser welding processes is further developed. During introducing the laser weld zone 7, for example in a sapphire-sapphire-enclosure 1 using crystalline samples, the upper substrate 4 may lift up and separate from the lower substrate 3. The gap that develops in between the substrates 3, 4 can still be hermetically sealed by means of the laser weld. However, if the laser weld zone 7 is not circumferentially closed around the enclosure 1, a gap may persist. When the end points of the laser weld zone 7 are defined such a gap can be embodied as channel 5, where the width can be defined during laser welding by means of the stopping points of the laser weld zone 7.

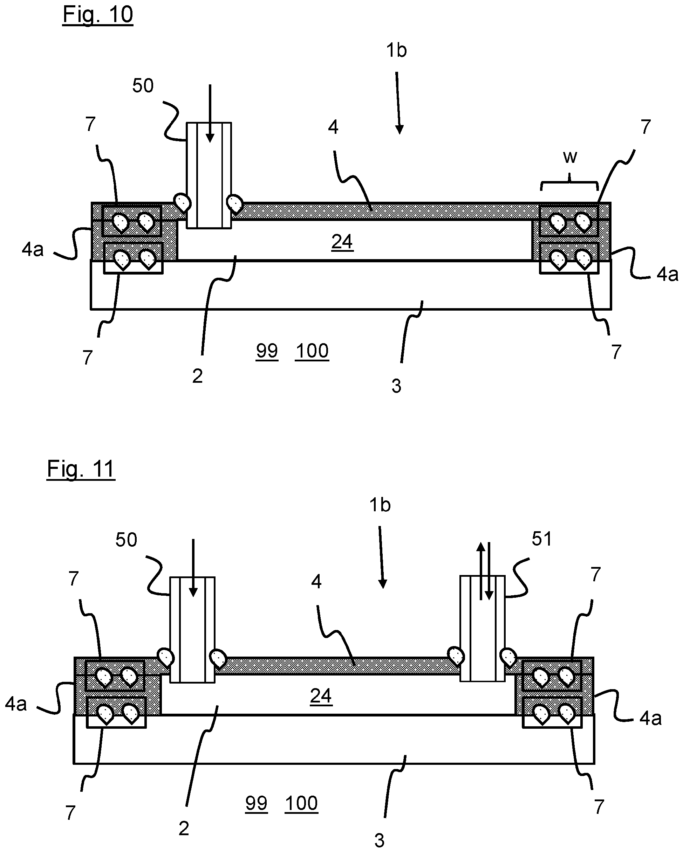

In a further development an enclosure 1 may comprise three substrate layers 3, 4, 4a or more to form the enclosure 1. The first substrate 3 functions as base layer below the cavity 2, the second substrate 4 functions as a top layer above the cavity 2, and an intermediate layer or intermediate substrate 4a is arranged in between those two substrates 3 and 4, see e.g. Fig. 10. The first substrate 3 may thus be welded to the intermediate substrate 4a and the second substrate 4 may be welded also to the intermediate substrate 4a by means of two planes of laser weld zones 7.

In forming the laser weld zone 7 each single shot of a laser pulse generates a nonlinear absorption. This zone may or may not later be visible as an absorptive volume (e.g. black spot). In the latter the term "nonlinear absorption zone" is used to denote the in-situ process volume, where the nonlinear absorption takes place and/or if some permanent modification to the linear absorption has happened, e.g. cases of visible damage in the substrate. This nonlinear absorption zone can cause heat accumulation, that takes place in the direction towards the laser pulse shot. Above the area of nonlinear absorption, which may correspond more or less with the laser focus and which may comprise a size of a few micrometers, an elongated bubble-shaped region may be formed, having only a few micrometers in width but typically up to several tens of micrometer in height (it is also referred to as "bubble" due to its typically quite characteristic shape comparable to an elongated bubble). Around that bubble-shaped region there is situated a melting region, wherein temperatures above Tg may be reached in the glass, which therefore has resolidified (after cooling or dissipation of warmth). The melting region with the included elongated bubble may usually clearly be identified, for example, with a light microscope, since its density and/or the refractive index has changed with respect to the surrounding material of the respective substrate (e.g. glass). In some cases, the area of nonlinear absorption can also be observed as optical damage on the lower tip of the melting region. Each laser spot and thus each laser weld line 6a, 6b, 6c may thus be identifiable, for example by optical means. One single laser spot may also be referred to as heating zone.

An immersible sensor element 1a for measuring fluid properties, such as blood pressure or blood glycose level, may comprise an enclosure 1 as described herein and a sensor element 21, in particular a fluid sensor element 21.

In Fig. 10 and Fig. 11 a cross sectional view in a vertical plane as defined by the arrows A and B of Fig. 4 of an immersible donator element 1b for changing or adapting fluid properties, in particular for emitting a pharmaceutical product or a pharmaceutical substance is shown.

The immersible donator element 1b preferably comprises an enclosure as described herein and at least one function zone in cavity 2 and comprising a donator fluid reservoir 24. In addition, an intermediate substrate 4a may be located between the at least first substrate 3 and second substrate 4 defining cavity 2 by means of a central cut out zone or central opening.

The immersible donator element 1b may further comprise a controllable valve 26 for adjusting the amount and/or time of pharmaceutical product to be emitted to outside 99. Outside 99 may comprise an outside fluid 100 surrounding the immersible donator element 1b.

Port 50 connects cavity 2 with a fluid source not explicitly shown in the drawings which may, when the immersible donator element1b is located within the body of a patient, be an extracoporal fluid source or may be an intracorporal fluid source for supplying a specific pharmaceutical substance to cavity 2. Due to a pressure in port 50 and the setting of flow regulator 26, the amount of pharmaceutical substance delivered to outside 99 and outside fluid 100 may be controlled in a defined manner.

Significantly, and especially in case of aggressive or toxic pharmaceutical substances, a beneficially defined dilution of the pharmaceutical substance may be obtained before delivered to outside 99. In case of a negative pressure in Port 50, outside fluid 100 may be sucked into cavity 2 and a defined dilution of the phameceutical substance and outside fluid 100 is provided in cavity 2. Subsequently, the diluted phameceutical substance may be delivered to outside 99 in a defined manner based on an increased pressure in port 50 and a corresponding setting of flow regulator 26. In this embodiment, cavity 2 as a whole may serve as reservoir 24.

In the embodiment shown in Fig. 11, two ports 50, 51 are connected to cavity 2 and may serve in case of port 50 as a fluid delivery and in case of port 51 as a fluid discharge. Then, cavity 2 may be flushed with a cleaning fluid after treatment of the patient's body with the pharmaceutical substance to support an exact timely limited treatment.

If via port 51 a further substance is delivered t cavity 2, this substance may include a further pharmaceutical substance or an activator for the fluid delivered via port 50 and a mixture of phameceutical substances and / or an activated pharmaceutical substance may be delivered to outside 99 and outside fluid 100, especially in a defined diluted and timely controlled manner. This embodiment may also assist in the delivery of short-lived therapeutics

These embodiments may be used in general to also reduce a toxic influence on the whole body of a patient by a respective pharmaceutical substance as a very exact and locally confined application is provided, especially in a timely exact defined manner.

It has to be understood that shape and size of ports 50 and 51 are only shown exemplarily and depend on the respective application intended to be realized.

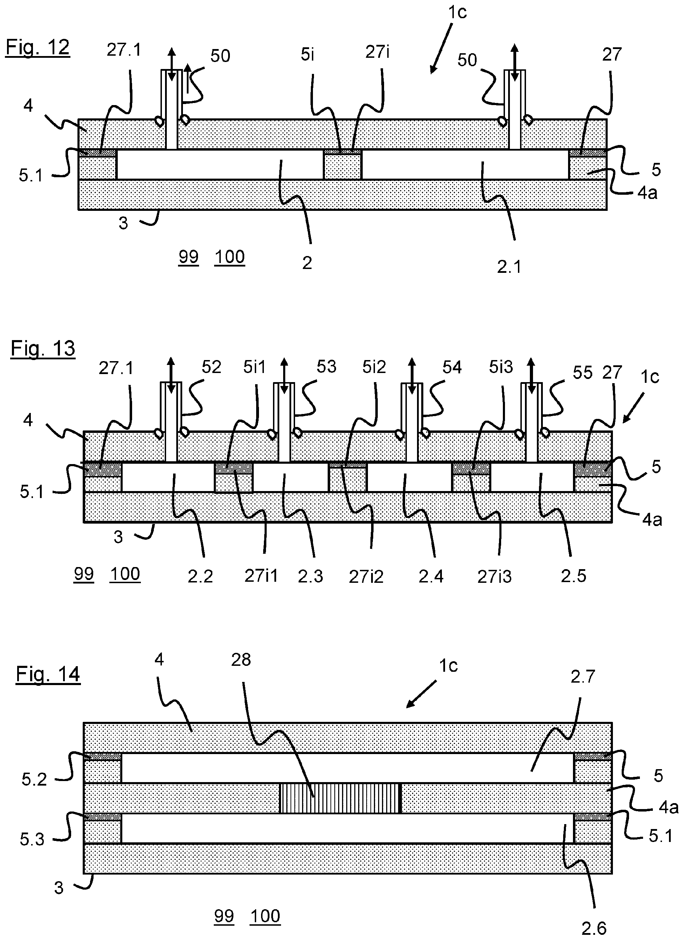

An immersible specific filter element 1c for being deployed in a fluid 99 such as blood as outside fluid 100, is shown in Figs. 12, 13 and 14 in a cross sectional view extending in a central vertical plane.

The immersible specific filter element comprises at least a first substrate 3 and a further substrate 4, 4a, wherein the first substrate is transparent at least in part and/or at least for a wavelength bandwidth; at least one common laser weld zone 7 wherein material of the first and further substrates is mixed , wherein the laser weld zone is designed such to permanently join the first substrate to the further substrate and/or to provide for an at least partially hermetic sealing between the first substrate and the further substrate; at least one function zone 2 comprising a cavity; at least a first fluid channel 5 allowing for a fluid inflow into said function zone from an outside 99, and at least a second fluid channel 5.1, 5.2, ... allowing for a fluid outflow into said outside 99, wherein the second fluid channel 5.1, 5.2, ... is designed such that selectable particles or components of the fluid are retained inside the cavity 2 when said fluid streams through said filter element.

The further substrate may comprise a second substrate 4 or in addition one intermediate substrates 4a or in further embodiments a plurality of intermediate substrates 4a.

Reference is made to figure 12, disclosing an immersible specific filter element 1c comprising two cavities 2 and 2.1 defined by respective cut out zones or openings in intermediate layer 4a. Each cavity comprises a channel 5, 5.1 allowing for a fluid flow between function zones in cavities 2, 2.1 and an outside 99, respectively. Outside 99 may comprise an outside fluid 100 surrounding the immersible specific filter element 1c. Between cavities 2 and 2.1 an intrinsic channel 5.i allows for a fluid flow between function zone of cavity 2 and function zone of cavity 2.1.

In a first embodiment, channels 5.1 and 5 comprise each at least a part-channel 5a, 5b ..., preferably provided in the form of one or more nanovoid(s), having a diameter Da or a height and a width of Da. In this first embodiment, channel 5i comprises at least a part-channel 5a, 5b ..., which also may be provided in the form of one or more nanovoid(s) based on a later intended use, having a diameter Df or a height and a width of Df with Df < Da.

In a further embodiment, Channels 5, 5.1, 5.i may also comprise each a filter element 27, 27.1 27i having a filter material, such as paper, porous glass, e.g. CoralPor^{®}, having an effective filter diameter in case of channels 5 and 5.1 of Da and in case of channel 5i of Df with Df < Da.

Alternatively, filter element 27 is arranged as a further intermediate layer between

In a further embodiment, via ports 50 a particle containing fluid may be delivered to cavity 2, 2.1 ensuring that particles of a certain size greater than Da remain in cavity 2, 2.1, whereas channel 5i allows for a fluid transport between cavity 2 and 2.1 to allow for a mixture of the fluids delivered via ports 50. If soluble particles will be delivered to captivity 2, 2.1 these particles remain in cavity 2, 2.1 until these particles are resolved to a size smaller than Da ensuring that only a defined maximum concentration of the pharmaceutical substance of these particles is delivered to outside fluid 100.

If a fluid is extracted from cavities 2. 2.1 via ports 50 then it is ensured that only particles having a particle size of less than Da are entering cavities 2, 2.1 as for instance upon a suitable choice of Da red and white blood cells may be excluded from entering cavities 2, 2.1 whereas blood serum and blood platelets may be collected in cavities 2, 2.1 and extracted via ports 50. Erythrocytes typically have a mean diameter in the range of about 7,5 µm and a mean thickness of 2 µm, leukocytes have typically a size of 7 to 20 µm, but blood platelets only a seize in the range of 1 to 4 µm. Accordingly choosing Da in a range from 5 µm to 6 µm may provide for the above mentioned separation. This filtering arrangement allows for a locally restricted and focused real time analysis of respective blood properties of blood without a cumbersome and time-consuming extraction as f.i.by means of syringes or other invasive medical devices.

In Figure 13 a filter cascade is shown as an embodiment of an immersible specific filter element 1c, comprising four cavities 2.2, 2.3, 2.4 and 2.5 each connected to a respective port 52, 53, 54 and 55. Cavities 2.2, 2.3, 2.4 and 2.5 are defined by cutout zones in intermediate substrate 4a.

In a first embodiment, channels 5.1 and 5 comprise each at least a part-channel 5a, 5b ..., which also may be provided in the form of one or more nanovoid(s) based on a later intended use, having a diameter Da or a height and a width of Da. In this first embodiment, each channel 5i1, 5i2, 5i3 comprises at least a part-channel 5a, 5b ..., which may be provided in the form of one or more nanovoid(s) based on a later intended use, having a respective diameter Df1< Da for channel 5i1, Df2< Da for channel 5i2 and Df3 < Da for channel 5i3 or a respective height and a respective width of Df1< Da for channel 5i1, Df2< Da for channel 5i2 and Df3 < Da for channel 5i3.

In a further embodiment, Channels 5, 5.1, 5.i1, 5i2, 5i3 may also comprise each a filter element 27, 27.1 27i having a filter material, such as paper, porous glass, e.g. CoralPor^{®}, having an effective filter diameter in case of channels 5 and 5.1 of Da and of Df1< Da for channel 5i1, Df2< Da for channel 5i2 and Df3 < Da for channel 5i3.

Based on the size of Df1, Df2 and Df3 complex mixtures of pharmaceuticals comprising soluble particles of a size greater than Df1, Df2 and/or Df3 complex mixtures may be obtained in cavities 2.2, 2.3,2.4 and 2.5 intermixing of which may be influenced by a positive or negative pressure in ports 52, 53, 54 and 55. A resulting mixture then may be released from cavity 2.5 via channel 5 and from cavity 2.2 via channel 5.1 to the outside fluid 100.

When fluid is extracted from cavities 2.2, 2.3, 2.4, 2.5 via ports 52, 53, 54 and 55, respectively filtered particles with a defined size are contained in these fluids depending on the size of Da, Df1, Df2 and/or Df3 allowing for a defined choice of particle sizes to be extracted.

From Figure 14 a two chamber embodiment of an immersible specific filter element 1c can be seen comprising two cavities 2.6, 2.7 having in case of cavity 2.7 channels 5 and 5.2 and in case of cavity 2.6 channels 5.1 and 5.3. In this embodiment an array of laser-shot nano-channels 28 is arranged in intermediate substrate 4a allowing for a laterally enhanced fluid exchange between cavities 2.7 and 2.8. The diameter of nano-channels may be chosen to provide a defined filtering effect between cavities 2.7 and 2.8.

Enclosure 1, immersible sensor element 1a, immersible donator element 1b or immersible specific filter element 1c, may be part of a microfluidic arrangement, especially within a complex analytic and / or therapeutic system which may at least in part be implanted into a patient's body for analytic and / or therapeutic purposes. However, the invention is not restricted to medical applications but may also be used in other environments, e.g. chemically harsh environments where materials as disclosed herein provide for an excellent fatigue strength.

Often, during production, a multitude of enclosures 1 comprising a common substrate, a plurality of common substrates or all substrates as common substrates is provided, each having a cavity 2 inside. The enclosures 1 are diced off, which is singulating each enclosure 1. So to say, a multitude of enclosures 1 may be prepared and provided at the same time with the same method step. Method steps of the method of manufacturing an enclosure 1 typically comprise Step A, wherein the substrates are aligned on top of each other, for example as substrates 3, 4, 4a also termed wafers 3, 4, 4a. A to be sealed component 21, 24, 26 may be arranged in Step A in each enclosure 1, if applicable. In Step B the wafers are stacked above each other in order to generate a waferstack, wherein several enclosures 1 will be obtained. The wafers 3, 4a, 4 may be optically aligned on top of each other. In a Step C of the method laser welding each cavity of the wafers 3, 4a, 4 is comprised. Step D could comprise separation or dicing, respectively, of the waferstack and thus singulating the several enclosures out of the waferstack.

It will be appreciated that the features defined herein in accordance with any aspect of the present invention or in relation to any specific embodiment of the invention may be utilized, either alone or in combination with any other feature or aspect of the invention or embodiment. In particular, the present invention is intended to cover an enclosure 1 configured to include any feature described herein. It will be generally appreciated that any feature disclosed herein may be an essential feature of the invention alone, even if disclosed in combination with other features, irrespective of whether disclosed in the description, the claims and/or the drawings.

It will be further appreciated that the above-described embodiments of the invention have been set forth solely by way of example and illustration of the principles thereof and that further modifications and alterations may be made therein without thereby departing from the scope of the invention.

### Reference list:

- 1: enclosure
- 1a: iimmersible sensor element for measuring fluid properties
- 1b: immersible donator element for changing or adapting fluid properties
- 1c: immersible specific filter element
- 2: function zone or cavity
- 2.1: second function zone or cavity
- 2.2: function zone or cavity
- 2.3: function zone or cavity
- 2.4: function zone or cavity
- 2.5: function zone or cavity
- 2.6: function zone or cavity
- 2.7: function zone or cavity
- 3: at least first substrate
- 4: second substrate
- 4a: further substrate, intermediate substrate or intermediate layer
- 5: trench / passage defining a channel, especially a fluid channel between the interior of enclosure 1 or cavity 2 and the outside 99 or exterior of enclosure 1 or cavity 2
- 5.1: at least a second fluid channel
- 5.2: at least a second fluid channel
- 5.3: at least a second fluid channel
- 5.4: at least a second fluid channel
- 5a: part-channel
- 5b: part-channel
- 5i: intrinsic channel
- 5i1: intrinsic channel
- 5i2: intrinsic channel
- 5i3: intrinsic channel
- 6a: first laser weld line
- 6b: second laser weld line
- 6c: third laser weld line
- 7: laser weld zone or weld line
- 8: ingrow preventing surface
- 11: edge of glass stack / enclosure
- 12: rim
- 15: contact area or welding plane
- 21: functional element
- 24: pressure difference generating means, reservoir
- 26: flow regulator
- 27: filter element
- 27i: filter element
- 27i1: filter element
- 27i2: filter element
- 27i3: filter element
- 28: laser-shot nano-channels
- 41: outer side of second substrate
- 43: outer side of first substrate
- 44: inner side of second substrate
- 46: inner side of first substrate
- 50: Port
- 51: Port
- 52: Port
- 52: Port
- 54: Port
- 55: Port
- 99: outside
- 100: outside fluid

- b: width of channel 5 in lateral direction perpendicular to the longitudinal extension of channel 5 and welding plane of weld line 7
- DF: distance of the at least one laser weld zone 7 and/or laser weld line 7 circumferencing the function zone 2 to the function zone 2
- HL: height of the welding zone in a direction perpendicular to its connecting plane
- HL1: height of the laser weld zone HL inside the first substrate
- HL2: height of the laser weld zone HL inside the second substrate
- h: height of channel 5 in a direction perpendicular to said welding plane
- w: width of the laser weld zone 7
- Δx: spacing between the last "shot" of the laser weld line and the beginning of the channel or trench 5 in lateral direction perpendicular to the longitudinal extension of channel 5

## Claims

1. Enclosure (1), comprising:
at least a first substrate (3) and a second substrate (4), wherein the first substrate is transparent at least in part and/or at least for a wavelength bandwidth;
at least one common laser weld zone (7) wherein material of the first and second substrates is mixed, wherein the laser weld zone is designed such to permanently join the first substrate to the second substrate and/or to provide for a hermetic sealing between the first substrate and the second substrate;
at least one function zone (2), for example comprising a cavity, enclosed inside the enclosure;
at least one fluid channel (5) allowing for a fluid flow between said function zone and an outside (99).

2. The enclosure (1) according to the preceding claim,
wherein the first substrate (3) comprises glass, glass ceramics, plastics or crystal such as sapphire, and/or
wherein the second substrate (4) comprises glass, glass ceramics, plastics, metal, silicon, or crystal such as sapphire, and/or
wherein the second substrate (4) and/or intermediate layer (4a) comprises a filter material, such as paper, porous glass, e.g. CoralPor^{®}, and/or
wherein said laser weld zone (7) is arranged in a welding plane (15) such that the welding plane cuts the laser weld zone e.g. centrical.

3. The enclosure (1) according to any of the preceding claims,
wherein said at least one fluid channel (5) is defined by the laser weld zone (7), for example by a shape or extension dimension of the laser weld zone, and/or
wherein said fluid channel (5) constitutes a trench in the laser weld zone (15).

4. The enclosure (1) according to any of the claims 2 or 3,
wherein said at least one fluid channel (5) is arranged in the welding plane (15), and/or
wherein said at least one fluid channel (5) comprises a height (h) in a direction perpendicular to said welding plane (15), which is smaller or equal to a height of the welding zone (7), and/or
wherein the laser weld zone (7) is situated with a height HL1 inside the first substrate (3) and with a height HL2 = h - HL1 inside the second substrate (4).

5. The enclosure (1) according to any of the preceding claims,
wherein said at least one fluid channel (5) comprises a flat or mostly rectangular shape, and/or
wherein said at least one fluid channel (5) comprises one or several ridge(s), and/or
wherein at least one fluid channel (5) is provided at opposing sides of the enclosure, for example one channel at every side of the enclosure, and/or
wherein said at least one fluid channel (5) is provided in the form of one or more nanovoid(s).

6. The enclosure (1) according to any of the preceding claims,
wherein the function zone (2) comprises said cavity, and wherein said cavity is connected to said fluid channel (5), and further comprising
a second cavity, wherein the second cavity is connected to a second fluid channel,
wherein the first cavity is connected to the second cavity by means of a passage.

7. The enclosure (1) according to the preceding claim,
wherein the passage (5) comprises a passage diameter and wherein the passage diameter is smaller than the diameter of the fluid channel and/or the second fluid channel.

8. The enclosure (1) according to any of the preceding claims,
wherein the at least one channel (5) comprises said height (h), for example in a direction perpendicular to said welding plane (15), of 10 nm or more, preferably 25 nm or more, further preferably 50 nm or more, further preferably 100 nm or more, or 300 nm or more, or even 500 nm or more, and/or
wherein the at least one channel (5) comprises a height (h), for example in a direction perpendicular to said welding plane (15), of 1000 nm or less, preferably 750 nm or less, further preferably 500 nm or less, further preferably 250 nm or less, or even 100 nm or less, and/or
wherein the at least one channel (5) comprises a width (b), for example in said welding plane (15), of at least 5 nm or more, further of at least 20 nm or more, preferably of 100 nm or more, further preferably of 1 µm or more, or even of 10 µm or more, or even 100 µm or more, and/or a width (b) of 2 mm or less, further of 500 µm or less, preferably 200 µm or less, further preferably 50 µm or less.

9. The enclosure (1) according to any of the preceding claims,
wherein said at least one fluid channel (5) provides for a helium leakage rate of 1×10⁻⁸ (mbar × liter)/s or more, preferably of 1×10⁻⁶ (mbar × liter)/s or more, and/or provides for a fluid flow rate of 5×10⁻⁶ (mbar × liter)/s or less, or 1×10⁻⁴ (mbar × liter)/s or less.

10. The enclosure (1) according to any of the preceding claims,
further comprising a pressure difference generating means (24), such as an expandable or shrinkable reservoir, an electromechanical pressing means, a chemical potential gradient source or a micro pump.

11. The enclosure (1) according to any of the preceding claims,
further comprising an ingrow preventing surface situated next to or around the at least one opening (5), the ingrow preventing surface, for example, comprising a biologically incompatible material and/or a smoothened and/or spiked surface.

12. The enclosure (1) according to any of the preceding claims,
further comprising a fluid property sensor (21) arranged on the outside (99) of the enclosure to be exposed in full fluid contact, and/or
further comprising at least one electrical connection from the function zone (2) to the outside (99) of the enclosure.

13. The enclosure (1) according to any of the preceding claims,
wherein the at least one laser weld zone (7) extends from within the first substrate (3) to within the second substrate (4) so that material from the first substrate is mixed into the second substrate and/or material from the second substrate is mixed into the first substrate to permanently and directly join the first substrate to the second substrate, and/or
wherein the first substrate (3) comprises an outer surface (43) and wherein the second substrate (4) comprises an outer surface (41), and wherein the at least one laser weld zone (7) comprises a first separation spacing S1 to the outer surface of the first substrate and a second separation spacing S2 to the outer surface of the second substrate, and/or
wherein in the laser weld zone (7) a convection zone is present, where material from the first substrate (3) is mixed with material from the second substrate (4).

14. The enclosure (1) according to any of the preceding claims, wherein the laser weld zone (7) is a portion of the first and/or second substrate (3, 4) having a refractive index that differs from a refractive index of a non-welded portion of the first and/or second substrate.

15. Enclosure (1) according to any of the preceding claims,
wherein the laser weld zone (7) is arranged around said function zone (2), for example circumferentially around said function zone, and/or
wherein the laser weld zone (7) is arranged such, that it comprises a third separation spacing S3 to the at least one opening (5), and/or
wherein at least one functional component (21) is arranged in said function zone (2), such as an electronics component, a MEMS, a MOEMS, or a medical dosage component, a medical sensor or a fluid property sensor.

16. Use of an enclosure (1) according to one of the preceding claims as a sensor element, a wafer level packaged component, a micro lens compound, a micro optical chip, an LED device, a laser diode device, a pharma packaging, a pharmaceutical product emitter, a blood property sensor.

17. Immersible sensor element (1a) for measuring fluid properties, such as blood pressure or blood glycose level, preferably comprising an enclosure according one of claims 1 to 15 and comprising:
at least a first substrate (3) and a second substrate (4), wherein the first substrate is transparent at least in part and/or at least for a wavelength bandwidth;
at least one common laser weld zone (7) wherein material of the first and second substrates is mixed, wherein the laser weld zone is designed such to permanently join the first substrate to the second substrate and/or to provide for a hermetic sealing between the first substrate and the second substrate;
at least one function zone (2), for example comprising a cavity, enclosed inside the enclosure, and comprising a sensor element (21), in particular a fluid sensor element (21); and
at least one fluid channel (5) allowing for a fluid flow between said function zone and an outside (99).

18. Immersible donator element (1b) for changing or adapting fluid properties, in particular for emitting a pharmaceutical product, preferably comprising an enclosure according one of claims 1 to 15 and comprising:
at least a first substrate (3) and a second substrate (4), wherein the first substrate is transparent at least in part and/or at least for a wavelength bandwidth;
at least one common laser weld zone (7) wherein material of the first and second substrates is mixed, wherein the laser weld zone is designed such to permanently join the first substrate to the second substrate and/or to provide for a hermetic sealing between the first substrate and the second substrate;
at least one function zone (2), for example comprising a cavity, enclosed inside the enclosure, and comprising a donator fluid reservoir (24);
at least one fluid channel (5) allowing for a fluid flow between said function zone and an outside (99).

19. Immersible donator element (1) as defined in the preceding claim,
Further comprising a controllable valve (26) for adjusting the amount and/or time of pharmaceutical product to be emitted.

20. Immersible specific filter element (1c), for being deployed in a fluid (99) such as blood, comprising:
at least a first substrate (3) and a further substrate (4, 4a), wherein the first substrate is transparent at least in part and/or at least for a wavelength bandwidth;
at least one common laser weld zone (7) wherein material of the first and further substrates is mixed, wherein the laser weld zone is designed such to permanently join the first substrate to the further substrate and/or to provide for an at least partially hermetic sealing between the first substrate and the further substrate;
at least one function zone (2) comprising a cavity;
at least a first fluid channel (5) allowing for a fluid inflow into said function zone from an outside (99), and at least a second fluid channel (5,1, 5,2, ...) allowing for a fluid outflow into said fluid (99), wherein the second fluid channel (5.1, 5.2, ...) is designed such that selectable particles or components of the fluid are retained inside the cavity 2 when said fluid streams through said filter element.
